# EUROPÄISCHE PATENTSCHRIFT

(11) **EP 0 999 797 B1**
(45) Veröffentlichungstag und Bekanntmachung des Hinweises auf die Patenterteilung: **03.12.2003**
(21) Anmeldenummer: 97936604.4
(22) Anmeldetag: 30.07.1997
(51) Int. Cl.: A61B 17/78, A61F 2/36, A61F 2/38

(54) **MARKNAGELSYSTEM ZUR FRAKTURHEILUNG BZW. KNOCHENVERLÄNGERUNG**
INTRAMEDULLARY OSTEOSYNTHESIS NAIL FOR HEALING FRACTURES OR BONE ELONGATION
SYSTEME DE CLOU D'OSTEOSYNTHESE INTRAMEDULLAIRE POUR LA GUERISON DE FRACTURES OU LE RALLONGEMENT D'OS

(43) Veröffentlichungstag der Anmeldung: 17.05.2000
(73) Patentinhaber: aap Implantate AG, 12099 Berlin (DE)
(72) Erfinder: AHRENS, Uwe, D-12107 Berlin (DE)
(74) Vertreter: Meissner, Peter E., Dipl.-Ing.
(86) Internationale Anmeldenummer: DE9701672
(87) Internationale Veröffentlichungsnummer: WO99005981

(56) Entgegenhaltungen:
- EP-A- 0 368 488
- WO-A-97/18776
- DE-A- 3 308 229
- DE-A- 3 537 318
- DE-A- 4 442 205
- DE-A- 19 619 093
- US-A- 5 549 610
- US-A- 5 620 445

## Beschreibung

Die Erfindung betrifft ein System, ausgehend von einem Marknagel, zur Verwendung bei allen menschlichen und tierischen Knochen zur Frakturheilung bzw.Knochenverlängerung, insbesondere zur Verwendung im Femurbereich.

Für die verschiedenen auftretenden Defekte sind jeweils bereits Einzellösungen bekannt. Beispielsweise werden Femurfrakturen mit Marknägeln versorgt. Bei einer Schenkelhalsfraktur kommen Schenkelhalsnägel zur Anwendung. Diese werden durch im Marknagel vorgesehene Bohrungen eingebracht und in den Schenkelhals eingeführt. Für den Knie- und Hüftbereich ist der Einsatz spezieller Prothesen bekannt. Bei einem Totalendoprothesenwechsel kann auf eine Revisionsprothese zurückgegriffen werden.
Mit den genannten Implantaten können im wesentlichen alle vorkommenden Frakturen versorgt und Knochenentfernungen überbrückt werden.

Dabei erweist sich allerdings als nachteilig, daß dem Patienten bei mehreren Frakturen evtl. verschiedene Arten von Implantaten eingesetzt werden müssen, die aber nicht zueinander kompatibel sind. Das kann im schlimmsten Fall dazu führen, daß bei einer Veränderung des Krankheitsbildes des Patienten ein kompletter Implantatwechsel notwendig ist.
Als nachteilig erweist sich weiterhin, daß die Anzahl der zu lagernden Teile bei dem Operateur hoch ist.

Die Schrift US-A-5.620.445 offenbart einen Marknagel, der an seinem distalen und proximalen Ende mit je einem Element über Schraub- oder Konusverbindungen vertängerbar ist.
Von den Verlängerungen trägt die eine Mittel zur Befestigung von Instrumenten zum Setzen und zur Extraktion des Marknagels.
Beide Verlängerungselemente sind mit Bohrungen zur Durchführung von Verriegelungsschrauben versehen.

Der Erfindung lag die Aufgabe zugrunde, ein Implantat zur Unterstützung bei Frakturheilung bzw. zur Überbrückung von Knochenverlusten bereitzustellen, das die oben genannten Probleme löst.

Die Aufgabe wird mit den Merkmalen des Anspruch 1 und Anspruchs 6 gelöst.
Vorteilhafte Weiterbildungen werden in den Unteransprüchen genannt.

Der Grundgedanke der Erfindung liegt darin, daß ein System, aufbauend auf einen Marknagel als Grundelement, vorgeschlagen wird, der wahlweise über Verbindungsmittel an seinem oberen und/oder unteren Ende mit vom Operateur für das jeweilige Krankheitsbild des Patienten individuell wählbaren und evtl. an ein verändertes Krankheitsbild anpaßbaren Komponenten kompatibel verbindbar ist, ohne daß im zweiten Fall ein kompletter Implantatwechsel notwendig ist.

Ausgehend von dem Marknagel kann somit bei Bedarf eine Erweiterung des Grundelementimplantates in den Knie- oder in den Hüftbereich erfolgen. Von großem Vorteil erweist sich hier, daß der Nagel auch die Verbindung mit neuen Komponenten bereits im implantierten Zustand gestattet, so daß eine Funktionserweiterung auch nach der Erstoperation ohne Aufwand eines kompletten Implantatwechsels möglich ist.

Vorteilhaft ist, daß das System als eine Art Baukastensystem, jederzeit im Bedarfsfall um weitere Bestandteile erweitert oder auch reduziert werden kann. Dies bringt zum einen verkürzte OP-Zeiten und Vorteile für den Patienten, da das System jeweils nach seinem Krankheitsbild, auch intraoperativ, individuell auszuwählen und bei Änderung des Krankheitsbildes anpaßbar ist.

Eine Anpassung des Implantates könnte beispielsweise dann notwendig werden, wenn bei Vorhandensein eines Tumors Teile des Knochens zu entnehmen sind, deren Größe aber erst während der Operation bestimmt wird. in Abhängigkeit von diesem Ergebnis kann der Operateur dann individuell entscheiden, welche Komponente auf den Marknagel aufzusetzen ist.

Vorteile der Erfindung bestehen auch darin, daß die Versorgung von Frakturen durch die Handhabung mit dem erfinderischen System als durchgängiges Instrumentarium vereinfacht und sicherer gestaltet wird.

Vorzugsweise handelt es sich bei dem Marknagel um einen Nagel mit äquidistanten Verriegetungsrinnen und kleinen Zielbohrungen über der gesamten Nagellänge und mit zwei großen Hilfsbohrungen am distalen und proximalen Ende. Dieses Hilfsbohrungen ermöglichen eine exakte Ausrichtung des Marknagels im Strahlengang eines Röntgengerätes und eine zeitweise Fixierung des Nagels, um eine entgültige Fixierung des Nagels im Knochen über Fixierungsstifte, die in die Rinnen eingeführt werden, zu erreichen. Eine Ausrichtung allein mittels Rinnen ist nur erschwert möglich, da im Gegensatz zu einer kreisförmigen Bohrung eine Rinne im Strahlengang immer als Rinne erscheint, unabhängig davon, ob Orthogonalität eingestellt ist oder nicht. Unter dem Begriff der Orthogonalität wird in diesem Zusammenhang verstanden, daß die Bohrung zu dem Strahlengang senkrecht ausgerichtet ist und somit als Vollkreis erscheint. Die außen liegenden Rinnen machen den Marknagel biegeweicher und gewähren somit eine bessere Krafteinleitung in den Knochen.

Der Marknagel als Grundelelement des erfindungsgemäßen Systems ist der anatomischen Form des Knochens angepaßt und kann wahlweise proximal oder distal implantiert werden.
Die Erfindung sieht vor, daß an beiden Enden des Marknagels Verbindungsmittel vorgesehen sind, um so einen größtmöglichen Indikationsbereich abzudecken, d.h. die Erweiterung des Grundelementes Marknagel sowohl den Knie- als auch in den Hüftbereich zu gewähren.
Diese Verbindungsmittel sind vorzugsweise als Innen- oder Außenkonus oder als Verschraubung ausgebildet.
Es ist aber durchaus möglich, daß der Marknagel ohne Anschluß von Komponenten als normaler Verriegetungsnagel verwendet wird.
Der Marknagel kann entfernt werden, indem eine stabförmige Vorrichtung auf ein Nagelverbindungsmittel aufgeschraubt oder aufgesetzt wird, und der Nagel dann aus dem Knochen nach Entfernen der Verriegelungsschrauben herausgezogen wird. Dabei ist darauf zu achten, daß beim Aufbringen der stabförmigen Entfemungsvorrichtung ein Gegenhalter vorhanden ist, um somit zu gewährleisten, daß keine Drehmomente in den Knochen übertragen werden.

Ein weiterer Aspekt der Erfindung sieht ein Trägerelement vor, das insbesondere als Komponente für das erfindungsgemäße System einsetzbar ist.
Dieses Trägerelement vereinigt mehrere sonst notwendige Einzelteile. Es dient zur Aufnahme und Fixierung beispielsweise eines Schenkelhalsnagels, der dann durch den Schenkelhals in den Femurkopf getrieben wird.

Das Trägerelement als spezielle Implantatkomponente für den Marknagel des Systems nach Anspruch 1 weist erfindungsgemäß ein oder mehrere Eintrittslöcher und jeweils mehrere Austrittslöcher auf, die zu jedem Eintrittsloch in einem geeigneten Winkel, vorzugsweise auf der gegenüberliegenden Seite, angeordnet sind, so daß der Nagel unter einem beim Einführen wählbaren Winkel durch das Trägerelement führbar ist. Das Trägerelement ist an seinem unteren Ende über ein Verbindungsmittel mit dem Marknagel verbindbar, vorzugsweise über eine Konusverbindung.
Bevorzugt weisen die Gänge für den Schenkelhalsnagel ein an den Nagel angepaßtes Profil auf, welches vorteilhafterweise nicht kreisförmig ist, um somit einer Rotation des Schenkelhalsnagels entgegenzuwirken.

Vorzugsweise sind jeweils drei Austrittslöcher in dem Trägerelement vorgesehen.
Ihre Mittelachsen können sich in einem Punkt schneiden. Das Winkelverhältnis des Eintrittsloches zu den Austrittslöchern kann so eingestellt sein, daß der Drehpunkt des einzuführenden Nagels mit dem Eintrittsloch zusammenfällt, er kann aber auch an anderen Punkten liegen.
Neben dem Trägerelement sind als weitere Komponenten für das erfindungsgemäße System verschiedene Prothesenelemente vorgesehen. Ein Beispiel dafür ist der hüftseitige Prothesenaufsatz. Er dient im Verbund mit dem Marknagel z.B. als Revisionsprothese.
Analog kann an dem unteren Nagelende eine knieseitige Prothesekomponente angesetzt werden. Diese dient ebenfalls im Verbund mit dem Marknagel z.B.als Revisionsprothese. Zur Überbrückung von größeren Defekten können auch beide Prothesekomponenten zusammen verwendet werden.
Letzlich kann ein Marknagel, z.B. bei Knochenverlust, durch ein gleichartiges Verlängerungsteil ergänzt werden.
Nachfolgend soll die Erfindung mittels eines Beispiels näher beschrieben werden.
Dabei zeigen:
Figur 1: einen Marknagel als Grundelement des erfindungsgemäßen Systems;
Figur 2: einen Teilausschnitt von Figur 1, mit einem Innenkonus als Verbindungsmittel, der an einem Verlängerungsteil angebracht sein kann;
Figur 3: ein Trägerelement mit hüftseitigem Kopf- und Schenkelhalsersatz;
Figur 4: das Trägerelement mit möglichen Positionen eines einzuführenden Schenkelhalsnagels und
Figur 5: einen Querschnitt eines Schenkelhalsnagels.

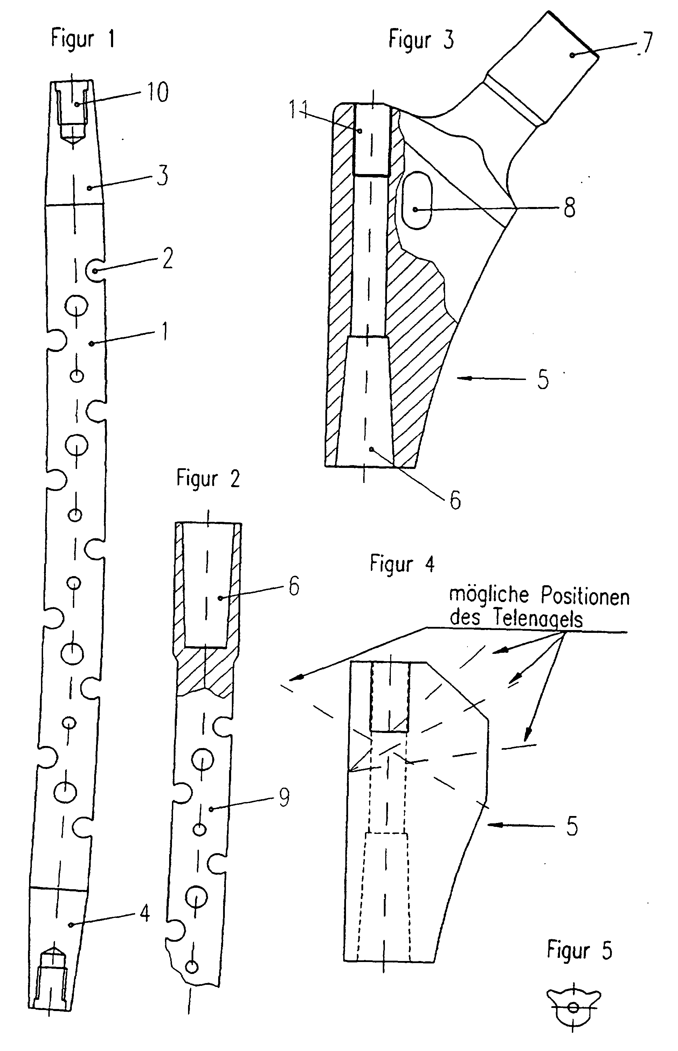

Figur 1 zeigt einen bekannten Marknagel 1, wie er bereits als Tibianagel eingesetzt wird. Beim Einsatz im Femurbereich ist er entsprechend größer gestaltet. Der Marknagel 1 weist außenliegende Rinnen 2 auf sowie kleinere und größere Bohrungen, wovon einige als Hilfsbohrungen zur Erleichterung der Ausrichtung des Marknagels im Strahlengang dienen. Das Grundelement Markangel weist an seinem oberen und unteren Nagelende Verbindungsmittel 3, 4 und 10 auf. Über diese Verbindungsmittel können die Komponenten kraft- und/oder formschlüssig aufgesetzt werden. Die hier gezeigten Verbindungsmittel sind als Konus oder Gewinde ausgebildet.
In Figur 2 ist als Verbindungsmittel ein Innenkonus 6 angegeben, der, an einem Verlängerungsteil 9 befindlich, auf den Außenkonus in Figur 1 aufsetzbar ist.
Figur 3 zeigt das Trägerelement 5, das mittels des Innenkonus 6 mit dem Marknagel 1 verbindbar ist. Das Trägerelement 5 ist mit einem hüftseitigen Prothesenaufsatz 7 versehen.
Das Langloch 8 soll eine Verrigelungsschraube aufnehmen, so daß die in die Komponente eingeleiteten Momente an den Knochen weitergeleitet werden können. Dies wirkt einer möglichen Schaftsprengung entgegen.
Figur 4 zeigt das Trägerielement 5 und verschiedenen Möglichkeiten des Durchführens eines Schenkelhalsnagels. Diese Nägel finden Verwendung zur Versorgung von Frakturen, die sich beispielsweise durch die beiden Rollhügel (pertrochantär) ziehen.
Der in das Trägerelement einzubringende Nagel ragt durch den Schenkelhals in den Femurkopf und stellt so einen Kragarm dar, der Biege- und Rotationskräfte in diesem Bereich aufnimmt. Das Trägerelement 5 zeigt ein Eintrittsloch und mehrere Austrittslöcher. Der Schenkelhalsnagel kann somit in einer der wählbaren Winkellagen gehalten werden.
Die Aussparungen des Trägerelementes weisen das gleiche Profil wie der jeweilige Nagel auf.
Figur 5 zeigt ein modifiziertes bekanntes Profil eines Schenkelhalsnagels.

## Patentansprüche

1. Marknagelsystem zur Frakturheilung bzw. Knochenverlängerung bei des Marknagelsystems menschlichen und tierischen Knochen, bestehend aus einem Marknagel (1) als Grundelement, der über Verbindungsmittel (3, 4, 6, 10 an seinem proximalen und/oder distalen Ende mit einem weiteren Element verbindbar ist, **dadurch gekennzeichnet dass** das weitere Element ein Trägerelement (5) ist, welches wahlweise
- ein oder mehrere Eintrittslöcher und ein oder mehrere Austrittslöcher aufweist, in die Nägel eingesetzt sind, wobei die Austrittslöcher zu den Eintrittslöchern eine derartige Lage haben, daß sich bei eingesetzten Nägeln ein unterschiedlicher Winkel zwischen der Nagellängsachse und der Marknagelachse ergibt, oder
- mit einem Prothesenaufsatz (7) zum Ersatz des Femuranteils des Hüftgelenks versehen ist, oder
- mit einem Prothesenaufsatz zum Ersatz des Femuranteils des Kniegelenks versehen ist, oder
- mit einer Marknagelverlängerung versehen ist.

2. Marknagelsystem nach Anspruch 1,
dadurch gekennzeichent,
daß die Verbindungsmittel des Marknagels als Konus (3,4,6) ausgebildet sind.

3. Marknagelsystem nach Anspruch 2,
**dadurch gekennzeichnet,**
**daß** es sich bei den Verbindungsmitteln um einen Innen- bzw. Außenkonus (3,4,6) handelt.

4. Marknagelsystem nach Anspruch 1,
dadurch gekennzeichent,
daß die Verbindungsmittel des Marknagels Gewinde (10, 11) sind.

5. Marknagelsystem nach Anspruch 4,
**dadurch gekennzeichnet,**
**daß** es sich bei den Verbindungsmitteln um ein Innen-(10,11) bzw. Außengewinde handelt.

6. Marknagelsystem nach einem der vorangegangenen Ansprüche,
**dadurch gekennzeichnet,**
**daß** das Trägerelement (5) an seinem dem Marknagel abgewandten Ende mit einem weiteren Verbindungsmittel (11) zur Verbindung mit weiteren Komponenten versehen ist.

7. Marknagelsystem nach einem der vorangegangenen Ansprüche,
**dadurch gekennzeichnet,**
**daß** im Trägerelement (5) jeder Durchtrittskanal für einen Nagel ein an den durchzuführenden Nagel angepaßtes Profil aufweist.

8. Marknagelsystem nach Anspruch 7,
**dadurch gekennzeichnet,**
**daß** es sich bei dem Nagel um einen Schenkelhalsnagel handelt.

9. Marknagelsystem nach einem der Ansprüche 1 oder 7 oder 8.
**dadurch gekennzeichnet,**
**daß** drei oder mehr Austrittslöcher für den Nagel vorgesehen sind.

10. Marknagelsystem nach einem der vorangegangenen Ansprüche,
**dadurch gekennzeichnet,**
**daß** an dem Trägerelement (5) ein Loch (8) zur Einbringung einer Verriegelungsschraube zur Übertragung von Momenten auf den Knochen vorgesehen ist.

11. Marknagelsystem nach Anspruch 10,
**dadurch gekennzeichnet,**
**daß** das Loch (8) ein Langloch ist.

## Claims

1. Intramedullary nail system for healing fractures or for bone elongation in human and animal bones, consisting of an intramedullary nail (1) as the basic element, which can be connected by connective means (3, 4, 6, 10) at its proximal and/or distal ends with another element of the intramedullary nail system,
**characterised in that**
the other element is a support element (5) which, optionally,
- has one or more entry holes and one or more exit holes, into which nails are inserted, the exit holes being positioned, in relation to the entry holes, in such a way that when the nails are inserted there is a variable angle between the lengthwise axis of the nail and the axis of the intramedullary nail, or
- has a prosthetic attachment (7) to replace the femoral section of the hip joint, or
- has a prosthetic attachment to replace the femoral section of the knee joint, or
- has an intramedullary nail extension.

2. Intramedullary nail system as in Claim 1,
**characterised in that**
the connective means of the intramedullary nail is in the form of a cone (3, 4, 6).

3. Intramedullary nail system as in Claim 2,
**characterised in that**
the connective means are an internal or external cone (3, 4, 6).

4. Intramedullary nail system as in Claim 1,
**characterised in that**
the connective means of the intramedullary nail are screw threads (10, 11).

5. Intramedullary nail system as in Claim 4,
**characterised in that**
the connective means are an internal (10, 11) or external screw thread.

6. Intramedullary nail system as in one of the preceding Claims,
**characterised in that**
the support element (5) has, at the end facing away from the intramedullary nail, another connective means (11 ) to connect it to other components.

7. Intramedullary nail system as in one of the preceding Claims,
**characterised in that**,
in the support element (5), each channel through which a nail passes has a profile which fits the nail that is to be taken through.

8. Intramedullary nail system as in Claim 7,
**characterised in that**
the nail is a femoral neck pin.

9. Intramedullary nail system as in one of Claims 1 or 7 or 8,
**characterised in that**
there are three or more exit holes for the nail.

10. Intramedullary nail system as in one of the preceding Claims,
**characterised in that**
there is, on the support element (5), a hole (8) for the introduction of a locking screw to transfer moments of force on to the bone.

11. Intramedullary nail system as in Claim 10,
**characterised in that**
the hole (8) is an elongated hole.

## Revendications

1. Système de clou médullaire pour la guérison de fractures ou le prolongement d'os pour êtres humains ou animaux, comportant un clou médullaire (1) comme élément de base, qui, par l'intermédiaire de moyens de liaison (3, 4, 6, 10) peut être relié à son extrémité proximale et/ou à son extrémité distale avec un autre élément dudit système de clou,
**caractérisé en ce que** ledit autre élément est un élément de support (5), qui au choix
- présente un ou plusieurs trous d'entrée et un ou plusieurs trous de sortie, dans lesquels sont introduits des clous, les trous de sortie ayant par rapport aux trous d'entrée une position telle qu'il en résulte pour les clous introduits un angle, entre l'axe longitudinal du clou et l'axe du clou médullaire, différent, ou
- est prévu avec une tête de prothèse (7) pour le remplacement de la partie du fémur de l'articulation de la hanche, ou
- est prévu avec une tête de prothèse pour le remplacement de la partie du fémur de l'articulation du genou, ou
- est prévu avec un prolongement de clou médullaire.

2. Système de clou médullaire selon la revendication 1,
**caractérisé en ce que** les moyens de liaison du clou médullaire sont conformés en cône (3, 4, 6).

3. Système de clou médullaire selon la revendication 2,
**caractérisé en ce que** lesdits moyens de liaison sont un cône mâle ou un cône femelle.

4. Système de clou médullaire selon la revendication 1,
**caractérisé en ce que** les moyens de liaison du clou médullaire sont des filetages (10, 11).

5. Système de clou médullaire selon la revendication 4,
**caractérisé en ce que** les moyens de liaison sont des filetages femelle (10, 11) ou mâle.

6. Système de clou médullaire selon l'une des revendications précédentes,
**caractérisé en ce que**, à son extrémité opposée au clou médullaire, l'élément de support (5) est pourvu d'autres moyens de liaison (11) pour la liaison à d'autres composants.

7. Système de clou médullaire selon l'une des revendications précédentes,
**caractérisé en ce que**, dans l'élément de support (5), chaque canal traversant pour un clou présente un profil adapté au trou traversant.

8. Système de clou médullaire selon la revendication 7,
**caractérisé en ce que** le clou est un clou pour col du fémur.

9. Système de clou médullaire selon l'une des revendications 1, 7 ou 8,
**caractérisé en ce que** le clou comporte trois ou plus trous de sortie.

10. Système de clou médullaire selon l'une des revendications précédentes,
**caractérisé en ce que**, sur l'élément de support (5), est prévu un trou (8) pour la mise en place d'une vis de blocage pour la transmission des moments aux os.

11. Système de clou médullaire selon la revendication 10,
**caractérisé en ce que** le trou (8) est oblong.
